# FASCICULE DE BREVET EUROPEEN

(11) **EP 1 043 962 B1**
(45) Date de publication et mention de la délivrance du brevet: **31.03.2004**
(21) Numéro de dépôt: 99900014.4
(22) Date de dépôt: 08.01.1999
(51) Int. Cl.: A61F 2/16

(54) **IMPLANT DE CHAMBRE ANTERIEURE POUR LE TRAITEMENT DE L'OEIL PHAQUE**
VORDERKAMMERIMPLANTAT ZUR BEHANDLUNG DES PHAKEN AUGES
ANTERIOR CHAMBER IMPLANT FOR TREATING THE PHAKIC EYE

(30) Priorité: 08.01.1998 FR 9800117
(43) Date de publication de la demande: 18.10.2000
(73) Titulaire: BAUSCH & LOMB INCORPORATED, Rochester, New York 14604-2701 (US)
(72) Inventeur: BAIKOFF, Georges, F-13008 Marseille (FR); HOFFMANN, Laurent, F-69003 Lyon (FR)
(74) Mandataire: Guerre, Dominique
(86) Numéro de dépôt international: PCT/IB1999/000014
(87) Numéro de publication internationale: WO 1999/034752

(56) Documents cités:
- EP-A- 0 346 245
- EP-A- 0 477 109
- EP-A- 0 563 602
- GB-A- 2 171 912
- US-A- 4 442 553
- US-A- 4 463 457
- US-A- 4 585 455
- BAIKOFF ET AL.: "Angle-fixated anterior chamber phakik intraocular lens or myopia of -7 to -19 diopters" JOURNAL OF REFRACTIVE SURGERY, vol. 14, no. 3, mai 1998, pages 282-93, XP002078963

## Description

La présente invention est relative à un implant oculaire de chambre antérieure, pour le traitement des affections de l'oeil phaque, notamment la myopie, l'astigmatisme, l'hypermétropie.

Un tel implant est, de manière bien connue, constitué d'une partie optique (lentille), généralement de contour circulaire et de section appropriée à la correction envisagée (plan, plan convexe, biconvexe, plan concave, biconcave, ménisqué, torique) ; à cette partie optique se raccorde une partie haptique destinée à fixer la position de l'optique dans la chambre antérieure. La partie haptique est constituée d'anses fines et déformables qui, en service, viennent se loger dans l'angle irido-cornéen.

Par exemple le document EP0346245 décrit décrit un tel implant selon l'art antérieur qui constitue le préambule de la revendication 1.

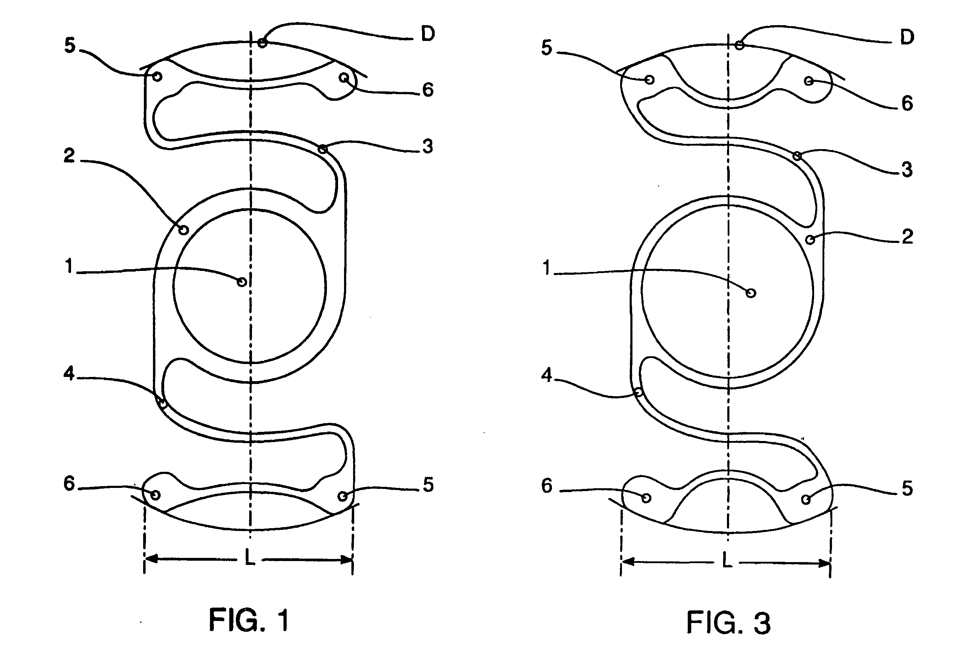

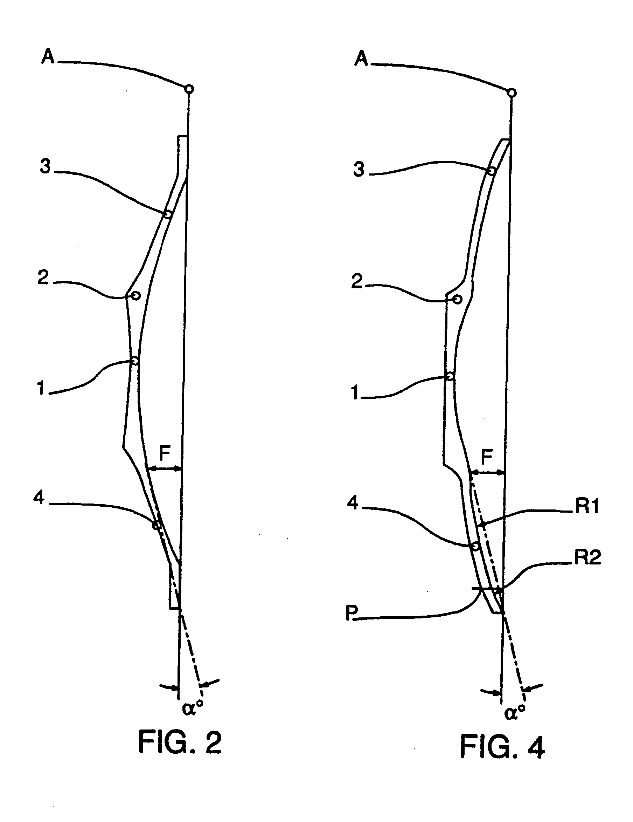

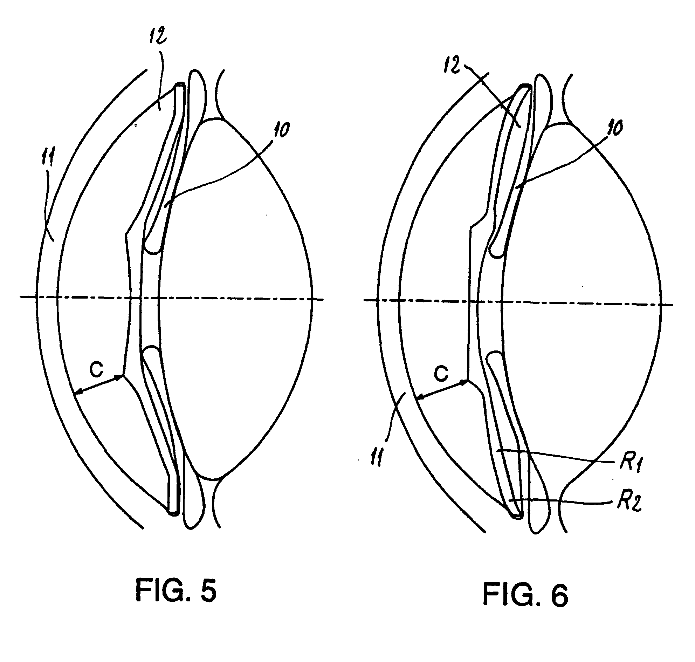

Un implant du genre ci-dessus, destiné à la correction de la myopie, est représenté schématiquement sur les figures 1, 2 et 5 annexées à la présente description, la figure 1 étant une vue en plan, la figure 2 une vue en élévation de profil, et la figure 5 une vue schématique de l'implant selon les figures 1 et 2 en position dans un oeil.

On voit sur la figure 1 la partie optique 1, la zone de raccordement 2 entre cette partie optique et les deux anses 3 et 4 qui sont de forme générale en S et dont la partie distale de chacune présente deux pieds d'appui 5 et 6.

On voit sur la figure 2 que la partie optique 1 est de type concave et que les anses 3, 4 sont angulées depuis leur partie proximale de raccordement à la partie optique 1 vers leur partie distale d'appui, par rapport à un plan vertical d'appui A.

Cet angle α est de l'ordre de 15° et est destiné à assurer que la partie optique 1 se trouve placée dans la chambre antérieure de l'oeil, sans risque de contact ni avec la cornée sur sa face avant, ni avec l'iris sur sa face arrière.

On voit également sur la figure 2 que l'extrémité distale des anses 3 et 4 se confond sur une longueur relativement importante avec le plan vertical A.

L'implant est d'une largeur d'environ 5,5 mm (référence L sur la figure 1) et s'inscrit dans une courbe enveloppe ayant un diamètre compris entre 12 mm et 13,5 mm (référence D sur la figure 1). Un tel implant est adapté à la plupart des formes et dimensions des yeux rencontrées chez les patients relevant de la chirurgie réfractive.

Cependant, avec les précédents modèles, on a constaté dans un nombre non négligeable de cas, des irritations dues au fait que la partie distale des anses vient en appui sur la périphérie de l'iris.

La présente invention a pour but de remédier à cet inconvénient en diminuant très fortement le risque d'appui de cette partie distale des anses sur la périphérie irienne, quelle que soit la taille de l'oeil dans lequel il est implanté.

Selon l'invention, chaque anse vue de profil présente un profil en arche à double rayon de courbure pour placer la surface d'appui dans l'angle irido-cornéen.

L'invention sera mieux comprise à l'aide de la description ci-après d'un exemple de réalisation non limitatif, en relation avec les figures 3, 4 et 6.

La figure 3 est une vue schématique en plan d'un implant myopique de chambre antérieure selon l'invention.

La figure 4 est une vue schématique en élévation de profil de l'implant représenté à la figure 3.

Et la figure 6 est une vue schématique de l'implant selon les figures 3 et 4 en position dans l'oeil.

Les références des parties qui correspondent à celles de l'implant illustré sur les figures 1 et 2 sont identiques. On y trouve une partie optique 1, une partie périphérique de raccordement 2 et deux anses 3 et 4 de forme générale en S (vues en plan) dont la partie distale présente des pieds d'appui 5 et 6.

Chaque anse présente, vue de côté selon figure 4, un profil en arche à double rayon de courbure. Le rayon de courbure (R1) de la partie proximale est plus grand que celui de la partie distale (R2). Le point de passage d'un rayon de courbure à l'autre se trouve (cf. repère P sur la figure 4), à une distance de l'extrémité distale de l'anse considérée, environ égale au quart de la hauteur de ladite anse, selon une direction parallèle au plan d'appui A.

L'angulation générale de l'anse par rapport au plan d'appui A est compris entre 13,5° et 16 °, et est de préférence de 15,12° (angle α sur la figure 4), pour un implant de 12,5 mm de courbe enveloppe.

La valeur des rayons de courbure est comprise entre 10 et 35 mm pour la partie proximale (R1), et de 2,5 mm pour la partie distale (R2).

Avec ces dispositions, la flèche de l'anse (référence F sur la figure 4) se trouvera environ à 1 mm du plan d'appui A et le point de l'optique le plus proche de la cornée se trouvera à 1,62 mm (référence c sur la figure 6). On garantit ainsi que les déformations des anses, secondaires à celles du globe oculaire, ne risquent pas de provoquer un contact de l'implant avec la cornée ou avec l'iris.

La forme en arche à double rayon de courbure assure que les anses ne seront en appui que sur les structures relativement résistantes de l'angle irido-cornéen (éperon scléral, trabéculum).

Plus précisément, conformément à la figure 6 et par différence à la figure 5 :
- la partie proximale de chaque anse, à grand rayon de courbure (R1), est quasiment parallèle au plan optique ; elle est située le plus loin possible de l'iris, pour permettre un jeu pupillaire sans contact entre l'anse et l'iris ;
- la partie distale de chaque anse, à petit rayon de courbure (R2) rejoint la partie proximale pratiquement dans le site d'implantation des anses, correspondant à l'angle de la chambre antérieure ;
- chaque anse se place ainsi dans une position médiane entre l'iris 10 et l'endothélium cornéen 11 ;
- la surface d'appui de chaque anse dans l'angle irido-cornéen demeure limitée ; pratiquement tout contact entre la partie distale de chaque anse et l'iris se trouve évité, en limitant toute réaction inflammatoire de ce dernier.

On a décrit ci-dessus un exemple de réalisation d'un implant dont la partie haptique comporte deux anses mais il va de soi que la présente invention est applicable à tout implant de chambre antérieure pour le traitement de l'oeil phaque, quel que soit le nombre d'anses de sa partie haptique.

## Revendications

1. Implant de chambre antérieure pour le traitement de l'oeil phaque, comportant une partie optique (1) se raccordant à des anses (3,4), **caractérisé en ce que** vue de profil chaque anse présente un profil en arche à double rayon de courbure, le rayon de courbure (R1) de la partie proximale étant plus grand que celui (R2) de la partie distale.

2. Implant selon la revendication 1, **caractérisé en ce que** le point de passage entre les deux rayons de courbure (R1) et (R2) est situé à une distance de l'extrémité distale de l'anse environ égale au quart de la hauteur de l'anse dans une direction parallèle au plan vertical A.

3. Implant selon la revendication 1 ou 2, **caractérisé en ce que** l'angulation (α) générale d'une anse est comprise entre 13,5° et 16° par rapport au plan d'appui (A).

4. Implant selon la revendication 3, **caractérisé en ce que** l'angulation d'une anse est de 15,12°, pour un implant de 12,5 mm de courbe enveloppe.

5. Implant selon l'une quelconque des revendications 1 à 4, **caractérisé en ce que** la valeur des rayons de courbure est :
R1 : ≥ 10 mm ≤ 35 mm
R2 : 2,5 mm

## Claims

1. Anterior chamber implant for treating the phakic eye, comprising an optical part (1) connected to loops (3, 4), **characterized in that**, viewed in profile, each loop has an arc-shaped profile with double radius of curvature, the radius of curvature (R1) of the proximal part being greater than that (R2) of the distal part.

2. Implant according to Claim 1, **characterized in that** the point of passage between the two radii of curvature (R1) and (R2) is situated at a distance from the distal end of the loop approximately equal to quarter the height of the loop in a direction parallel to the vertical plane A.

3. Implant according to Claim 1 or 2,
**characterized in that** the general angulation (α) of a loop is between 13.5° and 16° relative to the support plane (A).

4. Implant according to Claim 3, **characterized in that** the angulation of a loop is 15.12° for an implant with an envelope curve of 12.5 mm.

5. Implant according to any of Claims 1 to 4, **characterized in that** the value of the radii of curvature is:
R1: ≥ 10 mm ≤ 35 mm
R2: 2.5 mm

## Patentansprüche

1. Implantat der vorderen Augenkammer zur Behandlung des Linsenauges, umfassend einen optischen Abschnitt (1), der mit Schlingen (3, 4) verbunden ist,
**dadurch gekennzeichnet, dass**
jede Schlinge in Profilansicht ein Bogenprofil mit doppeltem Krümmungsradius aufweist, wobei der Krümmungsradius (R1) des proximalen Abschnitts größer als der Krümmungsradius (R2) des distalen Abschnitts ist.

2. Implantat nach Anspruch 1,
**dadurch gekennzeichnet, dass**
der Punkt des Übergangs zwischen den beiden Krümmungsradien (R1) und (R2) in einem Abstand vom distalen Ende der Schlinge liegt, der ungefähr dem Viertel der Höhe der Schlinge in einer Richtung entspricht, die parallel zur vertikalen Ebene A verläuft.

3. Implantat nach Anspruch 1 oder 2,
**dadurch gekennzeichnet, dass**
die allgemeine Winkelbildung (α) einer Schlinge in Bezug auf die Stützebene (A) zwischen 13,5° und 16° liegt.

4. Implantat nach Anspruch 3,
**dadurch gekennzeichnet, dass**
die Winkelbildung einer Schlinge für ein Implantat mit 12,5 mm Mantelkurve bei 15,12° liegt.

5. Implantat nach einem der Ansprüche 1 bis 4,
**dadurch gekennzeichnet, dass**
der Wert der Krümmungsradien wie folgt lautet:
R1: ≥ 10 mm ≤ 35 mm,
R2: 2,5 mm.
